# EUROPEAN PATENT APPLICATION

(11) **EP 4 253 519 A1**
(43) Date of publication of application: **04.10.2023**
(21) Application number: 21914947.3
(22) Date of filing: 27.08.2021
(51) Int. Cl.: C12M 1/26, B25J 13/00

(54) **CELL MOVING DEVICE**

(30) Priority: 28.12.2020 JP 2020219008
(71) Applicant: Yamaha Hatsudoki Kabushiki Kaisha, Iwata-shi, Shizuoka 438-8501 (JP)
(72) Inventor: ITO, Saburo, Iwata-shi, Shizuoka 438-8501 (JP)
(74) Representative: Müller-Boré & Partner Patentanwälte PartG mbB
(86) International application number: PCT/JP2021/031546
(87) International publication number: WO 2022/145086

(57) **Abstract**

A cell transferring device includes a head shiftable in three-dimensional directions and a controller that controls a shifting of the head. The head is to be attached with a tip including a leading end portion having a leading end opening for allowing a cell to enter and leave the tip, and includes a mechanism for sucking and discharging the cell into and from the tip through the opening. The controller executes a peeling-off control of causing the tip to peel off the cell adhered to a bottom surface of a well of a culture plate through the head. The controller controls, in the peeling-off control, the head in such a manner that the leading end portion of the tip performs a shifting containing a horizontal component at different positions along a vertical direction, the different positions including a position where the leading end portion comes into contact with the cell adhered to the bottom surface.

## Description

### Technical Field

The present invention relates to a cell transferring device that peels off and suck a cell adhered to a bottom surface of a container by using a suction tip and transfers the cell to a predetermined place.

### Background Art

The fields of chemical and biological researches involve, for example, an operation of transferring a single cell, a cell colony, or other cell, each referred to as "cell" simply in the description, from a culture plate where the cell is cultured to a working plate where the cell is examined and observed. The operation includes picking the desired cell from a well of the culture plate, and transferring and placing the picked cell to a well of the working plate.

The piking is manually executable by using a pipette to which a tip for sucking a cell is attached. However, a high skill is demanded to accurately peel off and suck the cell from a culturing surface like a bottom surface of the well. Patent Literature 1 discloses an organism picking device that automatically performs picking. The organism picking device is configured to perform the picking by shifting, in a horizontal direction, an organism picking tool in proximity to or in contact with a culturing surface and scraping a cell adhered to the culturing surface by the organism picking tool.

However, such a device that simply shifts the organism picking tool or the tip in the horizontal direction as described in Patent Literature 1 occasionally fails to peel off the cell from the culturing surface. It is considered from the studies by the present inventor that the main reason for the failure lies in existence of irregularities or protrusions and recesses each having a size larger than a thickness (of around several microns) of a cell on a well bottom surface serving as a culturing surface, or lies in a height difference in a bottom surface among a plurality of wells belonging to one plate.

For instance, even lowering a leading end portion of the tip to a certain height position and horizontally shifting the tip may fail to peel off the cell adhered to one recess of the bottom surface. If the tip leading end portion is lowered to fit in a deepest recess of the bottom surface, the tip leading end may be stuck on the bottom surface, and thus, the tip itself may fail to shift. The height position of the cell on the bottom surface is obtained from focus information about an observation image of the cell. However, an exact height position of the cell may be unobtainable due to an influence of an error attributed to an observation lens or an influence of a depth of field. In this case, a lowered position of the tip leading end portion is not accurately settable. This may result in a failure to peel off the cell.

### Citation List

### Patent Literature

Patent Literature 1: Japanese Patent Publication No. 5618810

### Summary of the Invention

An object of the present invention is to provide a cell transferring device that enables accurate picking of a cell by reliably peeling off the cell from a bottom surface of a container.

A cell transferring device according to one aspect of the present invention includes: a head that is to be attached with a tip including a leading end portion having an opening for allowing a cell to enter and leave the tip, includes a mechanism for sucking and discharging the cell into and from the tip through the opening, and is shiftable in three-dimensional directions; and a controller that controls a shifting of the head. The controller is configured to: execute a peeling-off control of causing the tip to peel off the cell adhered to a bottom surface of a cell container through the head; and control, in the peeling-off control, the head in such a manner that the leading end portion of the tip performs a shifting containing a horizontal component at each of different positions along a vertical direction, the different positions including a position where the leading end portion of the tip comes into contact with the cell adhered to the bottom surface.

### Brief Description of Drawings

Fig. 1 a diagram schematically illustrating an example of a configuration of a cell transferring device according to an embodiment of the present invention.
Fig. 2A is a cross-sectional view of a tip to be attached to a head, and illustrates a shifting mechanism and a suction mechanism of the tip.
Fig. 2B is a cross-sectional view of the tip in a suction action.
Fig. 2C is an enlarged view of a main portion shown in Fig. 2B.
Fig. 3 is a block diagram showing an electric configuration of the cell transferring device.
Fig. 4A and 4B are explanatory views explaining defects in a conventional cell peeling-off operation.
Fig. 5 shows steps A to F of a cell peeling-off operation.
Fig. 6A is a view illustrating a peeling-off operation of gradually shifting a tip leading end portion away from a bottom surface.
Fig. 6B is a view illustrating a peeling-off operation of causing the tip leading end portion to gradually approach the bottom surface.
Fig. 7 is a view illustrating another example of a cell peeling-off operation.
Figs. 8A to 8D are views each illustrating a specific example of a horizontal shifting of the tip in the cell peeling-off operation.
Figs. 9A to 9G are views each illustrates a specific example of a horizontal shifting of the tip in peeling off a cell which is a part of a cell colony.
Figs. 10A to 10E are views each illustrates another example of a horizontal shifting of the tip in the cell peeling-off operation.
Fig. 11 is a flowchart showing picking-up and transferring of the cell by using the cell transferring device.
Fig. 12 is a view illustrating another example of the cell peeling-off operation.
Figs. 13A to 13C are views illustrating another example of the cell peeling-off operation.
Figs. 14A to 14C are views illustrating a modification of the tip and a cell peeling-off operation adopting the modified tip.

### Description of Embodiments

Hereinafter, embodiments of the present invention will be described with reference to the accompanying drawings. A cell transferring device according to the present invention is adaptable to picking and transferring of an organism-based cell of various types. Examples of the organism-based cell include: a single cell, such as a blood cell and a singled cell; a small tissue medium, such as Histoculture; a cell aggregate, such as spheroid and organoid; an individual, such as zebrafish, nematodes, and a fertilized egg; and a two-dimensional or three-dimensional cell colony. The "cell" referred to in the present description cover all the types of these cells. In particular, the cell transferring device according to the present invention is suitable for picking and transferring of a cell generally required to be picked under microscopic observation, e.g., a single cell, a cell aggregate, and a cell colony. In the embodiment described below, a cell colony is mainly presumed to be picked. The cell colony is simply called a "cell C" in the detailed description as wel.

### [Overall configuration of a cell transferring device]

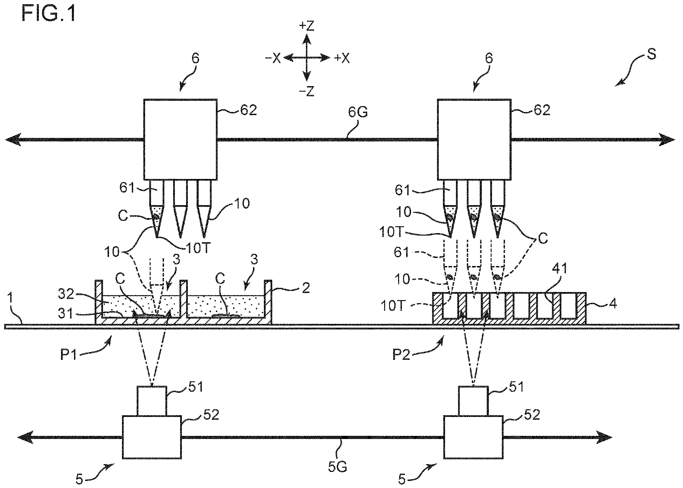

Fig. 1 a diagram schematically illustrating an overall configuration of a cell transferring device S according to an embodiment of the present invention. Fig. 1 exemplifies the cell transferring device S that transfers a cell C between two holders, that is, from a culture plate 2 to a destination plate 4 and vice versa. It is a matter of course that the cell transferring device S may be configured to transfer the cell C among three or more holders.

The cell transferring device S includes a base 1 having a horizontal placement surface and being transparent to light, a camera unit 5 located below the base 1, and a head unit 6 located above the base 1. The base 1 has a first placement position P1 where the culture plate 2 from which the cell C is transferred is placed and a second placement position P2 where the destination plate 4 to which the cell C is transferred is placed. The head unit 6 includes a plurality of heads 61 shiftable upward and downward in a Z-direction. Each head 61 has a lower end to which a tip 10 that sucks and discharges the cell C is attached. The camera unit 5 and the head unit 6 are movable in an X-direction (horizontal direction) and a direction (Y-direction) perpendicularly intersecting the sheet of Fig. 1. The culture plate 2 and the destination plate 4 are placed on an upper surface of the base 1 within a movable range of the head unit 6.

Generally, the cell transferring device S causes a plurality of tips 10 to respectively execute the picking of sucking, from the culture plate 2 where many cells C are cultured, the corresponding cells C. The device transfers the picked cells C to the destination plate 4 and discharges the cells C from the tips 10 to the destination plate 4 or wells 41. The camera unit 5 photographs each cell C held by the culture plate 2 before the picking of the cells C, and each of the cells C having good quality is selected to be transferred to the destination plate 4.

Hereinafter, each component of the cell transferring device S will be described. The base 1 is a rectangular flat plate having predetermined stiffness, a part of or a whole of the plate being made of light-transmissive material. The base 1 is preferably formed of a glass plate. Forming the base 1 with such light-transmissive material like the glass plate allows the camera unit 5 located below the base 1 to photograph the culture plate 2 and the destination plate 4 arranged on the upper surface the base 1 through the base 1.

The culture plate 2 has a plurality of wells 3 or cell containers, from which the cells C are transferred, for respectively culturing the cells C. Each of the wells 3 serves as a small container having an opening on the top thereof and a bottom surface 31 in a plate shape. The cultured cell C is adhered to the bottom surface 31. The well 3 contains a culture fluid 32 put therein. A test cell is seeded in the well 3, and cultured for a predetermined period to form a colony. The culture plate 2 is made of light-transmissive resin material or glass material to allow the camera unit 5 located below to photograph the cell C. For example, a commercially available 6-well plate, e.g., model number 3516 manufactured by Corning com., is adoptable as the culture plate 2.

The destination plate 4 has a plurality of wells 41 into which the cells C picked from the culture plate 2 are discharged. Each of the wells 41 includes a hole opening through an upper surface of the destination plate 4 and is defined by a bottom surface. One well 41 accommodates a necessary number of cells C (normally, one cell) together with the culture fluid. The cell C accommodated in the well 41 is subjected to: various tests including addition of a reagent, a reactant, or the like; observation; and culturation. The destination plate 4 is also made of light-transmissive resin material or glass material. For example, a commercially available 96-well plate, e.g., model number 3595 manufactured by Corning com., is adoptable as the destination plate 4.

The camera unit 5 captures an image of the cell C held by the culture plate 2 or the destination plate 4 from below, and includes a lens section 51 and a camera main body 52. The lens section 51 includes an objective lens for an optical microscope, and has a lens group for forming an image based on an optical image at a predetermined magnification, and a lens barrel accommodating the lens group. The camera main body 52 includes an imaging sensor, such as a CCD image sensor. The lens section 51 forms a image based on an optical image of a photographed object onto a light receiving surface of the imaging sensor. The camera unit 5 is movable in the X-direction and the Y-direction below the base 1 along a guide rail 5G extending leftward and rightward in parallel to the base 1. The lens section 51 is shiftable in the Z-direction for focusing.

The head unit 6 is provided to pick and transfer the cell C from the culture plate 2 to the destination plate 4. The head unit 6 includes the heads 61 and a head main body 62 to which the heads 61 are assembled. Each of the heads 61 has a distal end to which the corresponding suction tip 10 that sucks and discharges the cell C is to be attached. The head main body 62 holds the head 61 in a liftable and lowerable manner in the +Z-direction or upward and the -Z-direction or downward, and is movable in the +X-direction or horizontal direction and the -X-direction or horizontal direction along a guide rail 6G. The head main body 62 is shiftable in the Y-direction as well. In other words, the head 61 are shiftable or movable in three-dimensional directions X, Y, and Z.

### [Details of the tip and the head]

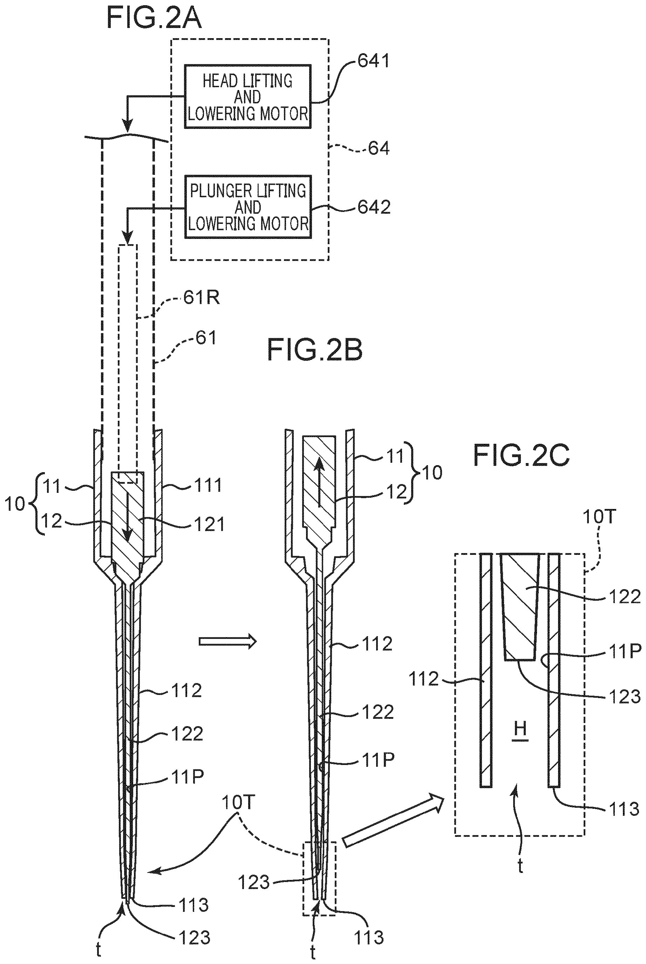

Fig. 2A is a cross-sectional view of the tip 10 to be attached to the head 61, and illustrates a shifting mechanism and a suction mechanism of the tip 10. The tip 10 serves as a tool that sucks and discharges the cell C to transfer the cell C, and includes a leading end portion 10T having a leading end opening t for allowing the cell C to enter and leave the tip. The tip 10 in the embodiment includes an assembly of a syringe 11 and a plunger 12. The syringe 11 has, in the inside thereof, a tubular passage 11P providing a suction route of the cell C. The plunger 12 advances or retracts in the tubular passage 11P while coming into sliding contact with an inner wall of the syringe 11 that defines the tubular passage 11P.

The syringe 11 has a syringe proximal end 111 in a cylindrical shape with a larger diameter, and a syringe main body 112 in a long cylindrical shape with a smaller diameter. The tubular passage 11P is defined by the syringe main body 112. The leading end opening t is provided on a syringe distal end 113 being a lower end of the syringe main body 112. The leading end opening t communicates with an end of the tubular passage 11P. The syringe proximal end 111 is continuous to another end of the syringe main body 112 via a tapered portion. The syringe proximal end 111 has an upper portion to be fitted to the lower end of the head 61.

The plunger 12 has a plunger proximal end 121 in a cylindrical shape, a plunger main body 122 in a needle shape continuous downward from the plunger proximal end 121, and a plunger distal end 123 being a lower end of the plunger main body 122. The plunger 12 is assembled to the syringe 11 in a state where the plunger proximal end 121 is accommodated in the syringe proximal end 111 and the plunger main body 122 is inserted in the tubular passage 11P of the syringe main body 112. The plunger distal end 123 protrudes out of the leading end opening t in a state where the plunger main body 122 is inserted deepest into the syringe main body 112 as shown in Fig. 2A. The plunger proximal end 121 has an upper portion to be attached with a rod 61R shiftable upward or downward in an inner space of the head 61.

The head main body 62 includes, in the inside thereof, a head drive part 64 serving as a shifting mechanism of shifting the tip 10 upward or downward with respect to the head 61 and serving as a mechanism for sucking and discharging the cell C into and from the tip 10 through the leading end opening t of the tip 10. The head drive part 64 has a head lifting and lowering motor 641 and a plunger lifting and lowering motor 642.

The head lifting and lowering motor 641 serves as a drive source for lifting and lowering the head 61 toward and away from the head main body 62. When the head 61 is driven by the head lifting and lowering motor 641 to be lifted or lowered, the tip 10 attached to the lower end of the head 61 is also lifted or lowered. That is to say, the leading end opening t of the leading end portion 10T is settable to a desired heigh position under an actuation control of the head lifting and lowering motor 641.

The plunger lifting and lowering motor 642 serves as a drive source for lifting and lowering the rod 61R in the inner space of the head 61. When the rod 61R is lifted or lowered, the plunger 12 attached to the rod 61R is also lifted or lowered. The lifting of the plunger 12 with respect to the syringe 11 generates a suction force at the leading end opening t. By contrast, the lowering of the plunger 12 with respect to the syringe 11 generates a discharge force at the leading end opening t. In other words, an action of sucking the cell C and an action of discharging the cell by the tip 10 are controllable under an actuation control of the plunger lifting and lowering motor 642.

Fig. 2A illustrates a state where the plunger 12 is lowered to a lowest position. The state represents a state prior to the sucking of the cell C, or a state immediately after the discharging of the cell C having been sucked into the tip 10. The plunger distal end 123 protrudes from the syringe distal end 113 slightly downward. Fig. 2B illustrates a state where the plunger 12 is lifted by a predetermined height. This state represents a state of the tip 10 in the suction action of sucking the cell C. Fig. 2C is an enlarged view of a main portion shown in Fig. 2B.

The plunger distal end 123 enters the inside of the tubular passage 11P in the suction action. At this time, a suction force is generated at the leading end opening t, and fluid around the leading end opening t is sucked into a suction space H coming into existence in the tubular passage 11P owing to the entering of the plunger distal end 123 into the inside. In other words, the culture fluid 32 enclosing the cell C is held in the suction space H. After the suction action, shifting the plunger 12 downward causes the fluid held in the suction space H to be discharged from the leading end opening t. A suction amount of the fluid is adjustable in accordance with a lifting height of the plunger 12, and a suction speed of the fluid is adjustable in accordance with a lifting speed of the plunger 12.

The tip 10 may be made of resin, metal, or glass. In this respect, at least one of the tip 10 or the culture plate 2 (well 3) is preferably made of an elastic deformable member. Although described later, the leading end portion 10T of the tip 10 may come into contact with the bottom surface 31 of the well 3 with a relatively strong force in the embodiment. Elastic deformation of either the tip 10 or the culture plate 2, or of both the tip and the culture plate can lead to a reduction in the risk of damage to theses members at the contacting. Besides, a strong sliding frictional force based on the elastic deformation force can act between the leading end portion 10T and the bottom surface 31 which are in contact with each other under an appropriate pressure. This consequently attains an enhanced force of peeling off the cell C from the bottom surface 31.

### [Electric configuration of the cell transferring device]

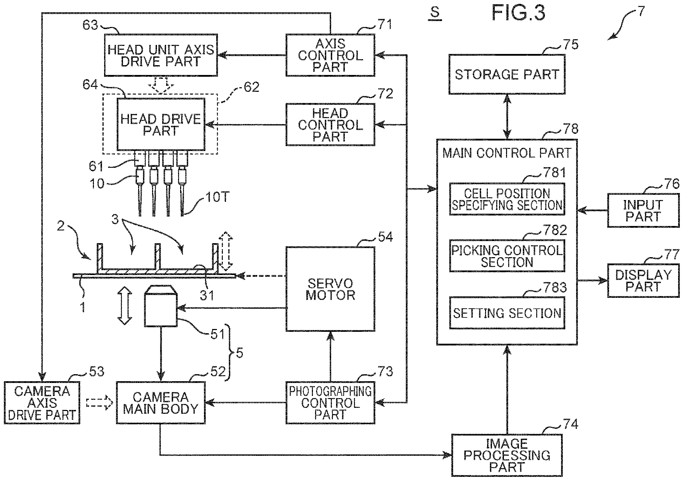

Fig. 3 is a block diagram showing an electric configuration of the cell transferring device S. The cell transferring device S includes a controller 7 that controls a shifting of the head unit 6 (see Fig. 1), and lifting and lowering of the head 61 (tip 10), i.e., a three-dimensional shifting of the head 61. The controller 7 additionally controls sucking and discharging of the cell C into and from the tip 10, and the movement of and photographing by the camera unit 5.

The cell transferring device S further includes a camera axis drive part 53, a servo motor 54, a head unit axis drive part 63, and a head drive part 64. The camera axis drive part 53 has a drive motor for horizontally moving the camera unit 5 along the guide rail 5G (Fig. 1). The servo motor 54 rotates forward or backward to shift the lens section 51 upward or downward at a predetermined resolution via an unillustrated power transmission mechanism. In this manner, a focus point of the lens section 51 is regulated to the cell C accommodated in the well 3. As denoted by a dashed line in Fig. 3, the base 1 may be moved upward or downward in place of the lens section 51. The head unit axis drive part 63 has a drive motor for horizontally moving or shifting the head unit 6 or the head main body 62 in the X-direction or the Y-direction along the guide rail 6G. The head drive part 64 is described above with reference to Fig. 2.

The controller 7 includes a microcomputer, and comes into effect to include a axis control part 71, a head control part 72, a photographing control part 73, an image processing part 74, a storage part 75, and a main control part 78 in response to execution of a predetermined program. An input part 76 that receives an input of various kinds of information to the controller 7, and a display part 77 that displays the various kinds of information are equipped. The input part 76 receives the input of the various kinds of operation information from an operator. In the embodiment, the input part 76 serves as a terminal that receives an input about a selection of a cell C. The display part 77 serves as a monitor that displays an image captured by the camera unit 5.

The axis control part 71 controls an operation of the head unit axis drive part 63. The axis control part 71 controls the head unit axis drive part 63 to move the head unit 6 to a predetermined target position along a horizontal direction. The axis control part 71 controls the head unit axis drive part 63 to realize: the movement of the head 61 or the tip 10 from the culture plate 2 to the destination plate 4 and vice versa; determination of a position of the head or the tip vertically above the cell C accommodated in the well 3; and determination of a position of the head or the tip vertically above the well 41 into which the cell is to be discharged. The axis control part 71 further controls the camera axis drive part 53 to control the movement of the camera unit 5 along the guide rail 5G. The axis control part 71 additionally controls a horizontal shifting of the head 61 in the peeling-off control of the cell C to be described later.

The head control part 72 controls the head lifting and lowering motor 641 of the head drive part 64 to lift or lower the head 61 to be controlled toward a predetermined target position. The head control part 72 further controls the plunger lifting and lowering motor 642 to generate a suction force or a discharge force at the leading end opening t of the tip 10 at a predetermined timing.

The photographing control part 73 controls photographing of the culture plate 2 or the destination plate 4 by the camera unit 5, e.g., a light exposure amount and a shutter timing. The photographing control part 73 further gives a control pulse to the servo motor 54 to shift the lens section 51 upward or downward at a predetermined pitch (e.g., tens of µm pitch) for focusing.

The image processing part 74 performs, onto image data acquired by the camera main body 52, image processing, such as edge detection and pattern recognition accompanied by feature amount extraction. The image processing part 74 executes, on the basis of an image of the culture plate 2 where the cell C is cultured, image recognition of existence of the cell C on the bottom surface 31. Similarly, the image processing part 74 executes, on the basis of an image of the well 41 to which the cell C has been transferred, recognition of the number of cells C accommodated in the well 41, the amount thereof, and the fluorescence intensity thereof.

The storage part 75 stores various set values, data, and programs about the cell transferring device S. The storage part 75 additionally stores information about the culture plate 2 to be used, e.g., data of a plate size, a size of the well 3, and a range of irregularities or protrusions and recesses on the bottom surface 31. The storage part 75 further stores setting information about a suction amount and a suction speed in the action of sucking the cell C, and a horizontal shifting pattern of the head and a shifting pitch thereof in the Z-direction in the peeling-off control.

The main control part 78 totally controls operations of the camera unit 5 and the head unit 6. The main control part 78 controls the camera unit 5 and the head unit 6 through the axis control part 71, the head control part 72, and the photographing control part 73 to: photograph the culture plate 2; perform picking of causing the tip 10 attached to the head 61 to suck the cell C selected to be transferred; and then transfer the cell C to the destination plate 4. In addition to the aforementioned total control, the main control part 78 further executes the peeling-off control of causing the tip 10 to peel off the cell C being a transfer target and adhered to the bottom surface 31 of the well 3 through the head 61.

The main control part 78 operably includes a cell position specifying section 781, a picking control section 782, and a setting section 783 for the peeling-off control. The cell position specifying section 781 acquires position information (XY-coordinate) on the cell C on the bottom surface 31 specified by the image processing part 74, and height or thickness information on the cell C. The picking control section 782 generates, on the basis of the setting information stored in the storage part 75, a control signal for each of a horizontal shifting, a vertical shifting, and a suction action of the head 61, and provides the generated signal to each of the axis control part 71 and the head control part 72. The setting section 783 sets information necessary for the peeling-off control depending on performance efficiency of the tip 10 to be used and the lens section 51.

### [Defects in peeling-off of the cell]

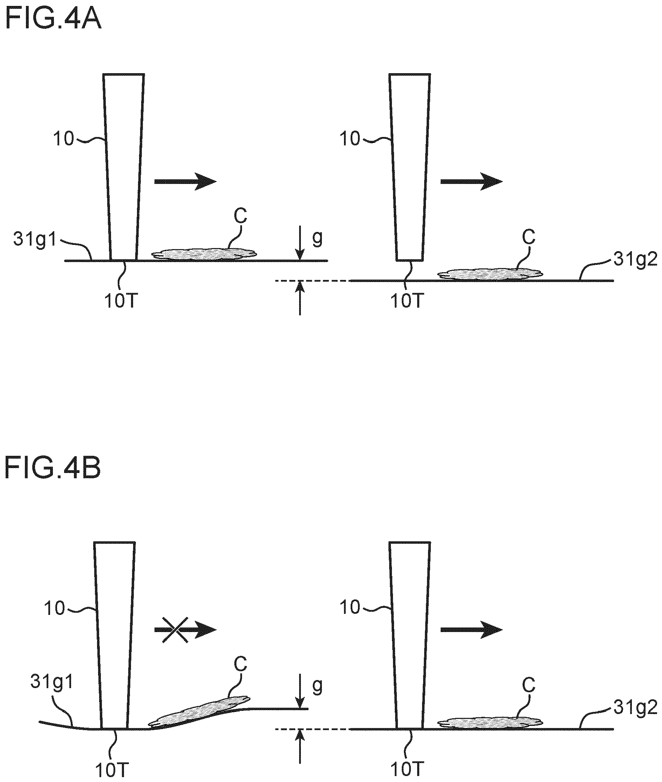

A cell seed placed and cultured in the culture plate 2 grows in a state of being adhered to the bottom surface 31 of the well 3 and results in forming a colony in many cases. Picking of the cell C in the form of the colony from the well 3 requires: peeling off of the cell C from the bottom surface 31 by the leading end portion 10T of the tip 10; and, subsequently, sucking of the peeled-off cell C into the tip 10 through the leading end opening t. In the peeling-off of the cell C, the tip 10 or the head 61 is shifted in a horizontal direction to allow the leading end portion 10T to scrape away the cell C in a state where the leading end portion 10T is in contact with the cell C adhered to the bottom surface 31. A defect in the peeling-off of the cell C will be described with reference to Figs. 4A and 4B.

The defect is that the bottom surface 31 of the well 3 has an unignorable height difference, i.e., surface irregularities or protrusions and recesses. The cell C to be picked has a thickness size of, for example, several microns. The bottom surface 31 is macroscopically seen as a horizontal plane, but has surface irregularities or protrusions and recesses on the order of several microns to tens of microns in micron order observation. Moreover, the present inventor has confirmed that a commercially available 6-well plate has a height difference of 90 µm at maximum among the six wells through measurement of the height of each bottom surface 31 of the corresponding well 3. In the existence of the height difference in the bottom surface 31, the leading end portion 10T of the tip 10 even lowered to a certain height position and shifted in a horizontal direction may fail to satisfactorily scrape away the cell C.

In the example illustrated in Fig. 4A, the well 3 has a first bottom surface 31g1 at a certain height position and a second bottom surface 31g2 at a position lower than the first bottom surface 31g1 by a height difference g. The first bottom surface 31g1 and the second bottom surface 31g2 constitute a bottom surface 31 of one well 3, or respectively belong to one well 3 and another well 3 included in a single culture plate 2. The height difference g is defined to be larger than the thickness of the cell C.

Under the condition, a lowering degree of the leading end portion 10T of the tip 10 is set to a height position of the first bottom surface 31g1, and a peeling-off operation of the cell C is executed. In this case, the cell C adhered to the first bottom surface 31g1 can be scraped away by the leading end portion 10T along with a horizonal shifting thereof. By contrast, the cell C adhered to the second bottom surface 31g2 located at a lower position than the leading end portion 10T that horizontally shifts cannot be scraped away.

Fig. 4B illustrates a state where a lowering degree of the leading end portion 10T of the tip 10 is set to a height position of the second bottom surface 31g2, and an operation of peeling off the cell C is executed. In this case, the cell C adhered to the second bottom surface 31g2 can be scraped away by the leading end portion 10T along with a horizonal shifting thereof. However, the lowering degree is too excessive for the first bottom surface 31g1, and thus the leading end portion 10T strongly hits the first bottom surface 31g1. Fig. 4B exemplifies a state where the first bottom surface 31g1 is pressed by the leading end portion 10T and curvingly deformed downward. In this case, the leading end portion 10T may be stuck on the first bottom surface 31g1 even in a horizontal shifting of the head 61, and thus, the leading end portion 10T may fail to horizontally shift. This results in making it difficult to reliably scrape away the cell C adhered to the first bottom surface 31g1.

Moreover, the depth of field of the lens section 51 of the camera unit 5 also has an influence on an accuracy of the peeling-off operation. The adhesion position of the cell C to the bottom surface 31 is obtainable from focusing position information on a two-dimensional image of the cell C photographed by the camera unit 5. For instance, a height position of the cell C is obtained on the basis of a height position of a body surface of the lens section 51 to a reference plane when a focused image of the cell C is acquired. However, the height position of the cell C obtained from the focusing position information involves an error attributed to the depth of field of the lens section 51. The error may lead the leading end portion 10T to a failure to hit or come into contact with the cell C in the peeling-off operation. The peeling-off control according to the embodiment aims at solve the failure.

### [Basic execution of the peeling-off control]

Subsequently, basic execution of the peeling-off control according to the embodiment will be described. In the peeling-off control, the picking control section 782 of the main control part 78 controls the head 61 to perform a shifting containing a horizontal component at different positions along a vertical direction. Each of the different positions along the vertical direction is desired to be a position where the leading end portion 10T of the tip 10 comes into contact with the cell C adhered to the bottom surface 31. However, the leading end portion 10T does not necessarily come into contact with the cell C at all the positions, and it is only required that the leading end portion 10T comes into contact with the cell C at least at one of the positions set by the picking control section 782 along the vertical direction. In other words, the "different positions along the vertical direction" may include a position where the leading end portion 10T comes into contact with the cell adhered to the bottom surface 31.

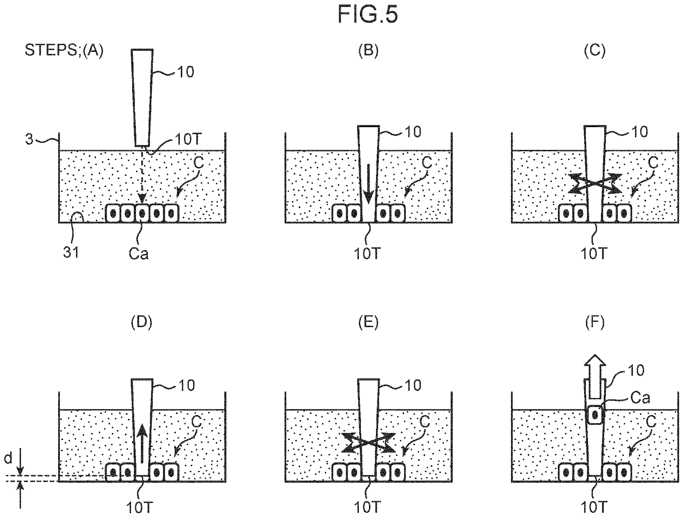

Steps A to F shown in Fig. 5 show a sequence of the operation of peeling off the cell C. The drawing illustrates a state where the cell C cultured in the form of a colony is adhered to the bottom surface 31 of one well 3 of the culture plate 2. Step A includes aligning the leading end portion 10T of the tip 10 with a cell fragment Ca to be picked, i.e., with a part of the cell C to be cut off therefrom, above the cell C. In the alignment, the axis control part 71 drives the head unit axis drive part 63 under the control of the picking control section 782 on the basis of XY-coordinate information on the cell fragment Ca specified by the cell position specifying section 781 to shift the head unit 6 in the XY-directions.

Step B includes lowering the leading end portion 10T of the tip 10 to a first position along the vertical direction. Exemplified here is a position as the first position where the leading end portion 10T comes into contact with the bottom surface 31. In the lowering, the head control part 72 drives the head lifting and lowering motor 641 of the head drive part 64 under the control of the picking control section 782 to lower the head 61 in the vertical direction. The leading end portion 10T penetrate the cell C through the lowering, resulting in making a cut around the cell fragment Ca.

When a height position of the bottom surface 31, i.e., a height position of the cell C, is obtained from the focusing position information on an image captured by the camera unit 5 in the selection of the position where the leading end portion 10T comes into contact with the bottom surface 31 as the first position, the head 61 is lowered in consideration of the above-described depth of field. Specifically, the height position of the cell C may involve an error attributed to the depth of field, and hence, the picking control section 782 causes the head lifting and lowering motor 641 to lower the head 61 by a distance obtained by adding the depth of field to the height position of the cell C specified by the cell position specifying section 781. By contrast, when the height position of the cell C is not recognized, the head 61 is lowered 61 by a distance obtained by adding a height difference in the bottom surface 31 actually measured in advance or acquired with various original values. In these cases, the tip 10 or the bottom surface 31, or both may elastically deform when coming into contact with each other.

Step C includes causing the tip 10 to perform a shifting (first horizontal shifting) containing a horizontal component at the first position. In the shifting, the axis control part 71 drives the head unit axis drive part 63 under the control of the picking control section 782 to shift the head unit 6 in the XY-directions, thereby causing the head 61 to perform a shifting containing a horizontal component. The shifting may cause bending of the tip 10. A pose time of withholding a start of the subsequent step may be provided for a period of several tens to hundreds of milliseconds after the execution of step C to wait for restoration from the bending. The execution of step C contributes to the peeling-off of the cell fragment Ca from the bottom surface 31 by causing the leading end portion 10T to shift the cell fragment Ca relative to the bottom surface 31.

Step D includes lifting the leading end portion 10T of the tip 10 from the first position to a second position along the vertical direction. A lifting pitch d is appropriately set in accordance with the thickness size of the cell C, and may be set within a range of, for example, the pitch d = several µm to 10 µm. In the lifting, the head control part 72 drives the head lifting and lowering motor 641 to lift the head 61 in the vertical direction by the pitch d.

Step E includes causing the tip 10 to perform a shifting (second horizontal shifting) containing a horizontal component at the second position. The operation of the mechanism is the same as in step C. Steps C to E may be repeated until the leading end portion 10T of the tip 10 is lifted by the height difference in the bottom surface 31. As described above, steps C to E can be repeated by 90 µm/d times in the existence of the height difference of 90 µm in the bottom surface 31. This attains reliable peeling-off of the cell fragment Ca even when the cell C is adhered to the bottom surface 31 having the height difference at any height position thereof. The number of repetitions of performing steps C to E may be appropriately set in consideration of a time required for the head 61 or the tip 10 to move from the culture plate 2 to the destination plate 4.

Step F includes causing the tip 10 to suck the cell fragment Ca peeled off from the bottom surface 31 thereinto. Execution of steps C to E makes the cell fragment Ca peeled off from the bottom surface 31 have a floating ability. A suction force is generated at the leading end portion 10T of the tip 10 in this state to allow the cell fragment Ca to be sucked through the leading end opening t (Fig. 2) and held in the tip 10. In the sucking, the head control part 72 drives the plunger lifting and lowering motor 642 of the head drive part 64 to lift the plunger 12, thereby generating the suction force. The cell fragment Ca held in the tip 10 is transferred to the destination plate 4 by the movement of the head unit 6 along the guide rail 6G. Thereafter, the cell fragment Ca is discharged from the tip 10 into a designated well 41.

### [Specific example of the peeling-off control]

Hereinafter, a specific example of the peeling-off will be described. Examples of the "shifting containing a horizontal component" performed at different positions in the peeling-off control include a linear shifting in a horizontal direction of the head 61, a curvy shifting, a linear and curvy shifting in combination, a circular shifting, and a swirl shifting. The shifting may contain a vertical component as long as the shifting contains the horizontal component. Examples of the shifting may include a swaying shifting of the leading end portion 10T like a pendulum, a spiral shifting thereof, and a vibrative shifting of the leading end portion 10T.

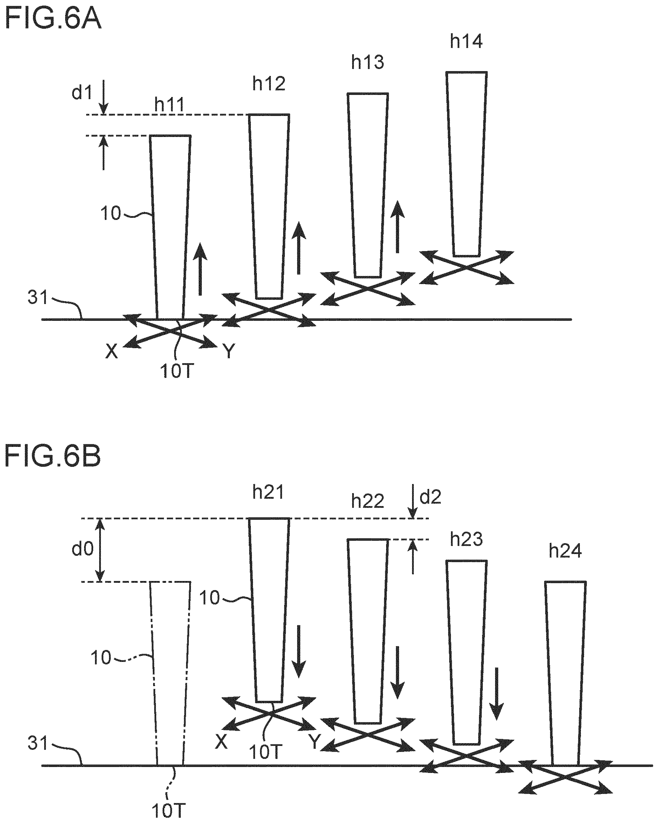

Fig. 6A is a view illustrating a peeling-off operation of gradually shifting the leading end portion 10T of the tip 10 away from bottom surface 31. This aspect is equivalent to the aspect of the peeling-off operation exemplified in Fig. 5. First, a position where the leading end portion 10T comes into contact with the bottom surface 31 is defined as a first position h11 along the vertical direction. At the first position h 11, a first horizontal shifting of horizontally shifting the head 61 linearly in an X-direction and a Y-direction is performed. Here, the first horizontal shifting is not limited to the linear horizontal shifting, and may be another horizontal shifting as mentioned above.

Subsequently, the head 61 is lifted in the vertical direction so that the leading end portion 10T reaches a second position h 12 which is higher by a pitch d1. It is clearly seen that the second position h12 is different from the first position h11 along the vertical direction and is away from the bottom surface 31 further than the first position h11. At the second position h12, a second horizontal shifting of horizontally shifting the head 61 linearly in the X-direction and the Y-direction is performed. That is to say, exemplified here is the second horizontal shifting executed in the same manner as the first horizontal shifting. In this case, the shifting control of the head 61 can be simplified. Of course, the first horizonal shifting and the second horizontal shifting may be executed in different manners from each other.

Thereafter, a third horizontal shifting is executed at a third position h13 to which the leading end portion 10T is further lifted by a pitch d1, and a fourth horizontal shifting is executed at a fourth position h14 to which the leading end portion is still further lifted by another pitch d1 . Although the example shows that each of the third horizontal shifting and the fourth horizontal shifting is a linear horizontal shifting in the XY-directions like the first horizontal shifting, each shifting may be another horizontal shifting different from the first horizontal shifting. When the shifting at each of the first position h11 to the fourth position h14 cannot cover the height difference in the bottom surface 31, the leading end portion is further lifted by a pitch d1, and another horizontal shifting of the head is executed. The horizontal shifting of the head 61 performable at more positions along the vertical direction achieves more reliable peeling-off of the cell C from the bottom surface 31 even having large irregularities or protrusions and recesses.

Here, the first to fourth horizontal shifting of the head 61 may not be necessarily executed at each position in this order of the first position h11 to the fourth position h14. For instance, the head 61 may be horizontally shifted in order of the first position h11, the third position h13, the second position h12, and the fourth position h14. Besides, the horizontal shifting may not be necessarily performed at each of the first position h11 to the fourth position h14, but may be performed during the shifting of the head 61 in the vertical direction.

In the peeling-off control illustrated in Fig. 6A, first, the first horizontal shifting is performed at the first position h11 closest to the bottom surface 31, and subsequently, the head 61 is shifted away from the bottom surface 31 in the vertical direction to execute the second horizontal shifting. The first position h11 indicates a position where the leading end portion 10T of the tip 10 comes into contact with the bottom surface 31. This consequently achieves execution of the first horizontal shifting in a state where the leading end portion 10T is ensured to be in contact with the cell C, and attains an increased ability of peeling off the cell C. The first position h 11 may be defined as a reference, and then, the second position h12 to the fourth position h14 for the subsequent horizontal shifting of the head 61 along the vertical direction may be determined on the basis the reference. That is to say, the position of the leading end portion 10T can be rapidly determinable, and the picking of the cell C is efficiently and speedily executable.

Fig. 6B is a view illustrating a peeling-off operation of causing the leading end portion 10T of the tip T10 to gradually approach the bottom surface 31. In this example, a position where the leading end portion 10T is away from the bottom surface 31 by a distance d0 is defined as a first position h21. The distance d0 corresponds to, for example, a height difference in the bottom surface 31. At the first position h21, a first horizontal shifting of horizontally shifting the head 61 linearly in the X-direction and the Y-direction is performed.

Next, the head 61 is lowered in the vertical direction so that the leading end portion 10T reaches a second position h22 which is lower by a predetermined pitch d2. It is clearly seen that the second position h22 is closer to the bottom surface 31 than the first position h21. At the second position h22, a second horizontal shifting of horizontally shifting the head 61 linearly in the X-direction and the Y-direction is performed. Thereafter, in the same manner as described above, a third horizontal shifting is executed at a third position h23 to which the leading end portion 10T is lowered by a pitch d2, and a fourth horizontal shifting is executed at a fourth position h24 to which the leading end portion is further lowered by another pitch d2. This aspect enables the leading end portion 10T to perform the operation of scraping away the cell C while causing the leading end portion 10T to gradually approach the cell C adhered to the bottom surface 31.

For the peeling-off operation illustrated in Fig. 6B, the leading end portion 10T may be once brought into contact with the bottom surface 31 as illustrated in the drawing before the leading end portion 10T is arranged at the first position h21. In the aspect shown in Fig. 6B, it is difficult to set a position to be the first position h21 due to an ungraspable actual distance from the height position of the leading end portion 10T to the bottom surface 31. By contrast, bringing the leading end portion 10T into contact with the bottom surface 31 in advance achieves detection of the height position from the bottom surface 31. In this manner, the first position h21 is easily settable.

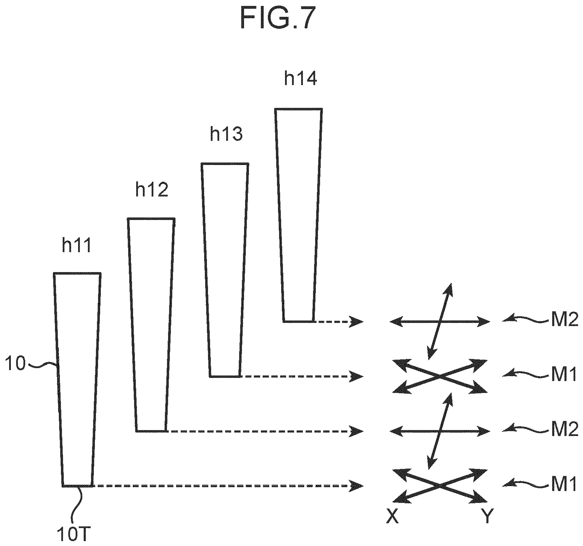

Fig. 7 is a view illustrating another example of the cell peeling-off operation. The example illustrated in Fig. 7 shows a peeling-operation of gradually shifting the leading end portion 10T away from the bottom surface 31 in a manner similar to Fig. 6A, but differs therefrom in shifting loci of the head 61 at height positions along the vertical direction. A first horizontal shifting at a first position h11 includes execution of a first shifting pattern M1 of shifting the head 61 in such a manner that the leading end portion 10T horizontally shifts linearly in an X-direction and a Y-direction. By contrast, a second horizontal shifting at a second position h12 includes execution of a second shifting pattern M2 of linear horizontal shifting in a diagonal direction at 45 degrees from the X-direction and a diagonal direction at 45 degrees from the Y-direction.

A shifting locus of the leading end portion 10T in the first shifting pattern M1 and a shifting locus of the leading end portion 10T in the second shifting pattern M2 intersect on an axially viewed plane of the tip 10. In the example illustrated in Fig. 7, a third horizontal shifting at a third position h13 adopts the first shifting pattern M1, and a fourth horizontal shifting at a fourth position h14 adopts the second shifting pattern M2. Specifically, the shifting locus of the leading end portion 10T is changed alternately between the first shifting pattern M1 and the second shifting pattern M2 per one-pitch lift of the leading end portion 10T. The different shifting loci of the leading end portion 10T described above allow directionally different peeling-off forces to act on the cell C adhered to the bottom surface 31. Consequently, the cell C can be more easily peeled off.

### [Specific example of the horizontal shifting of the tip]

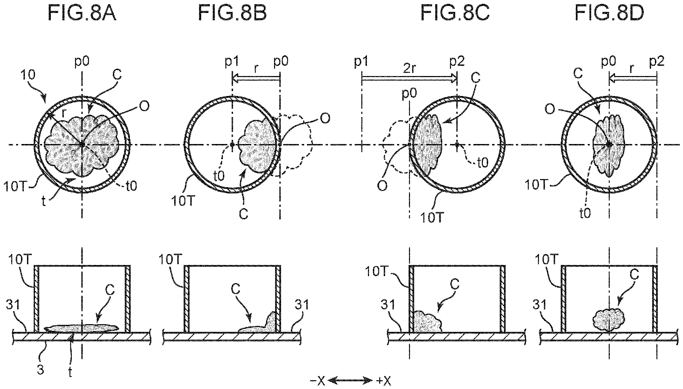

Next, the horizontal shifting of the tip 10 configured to shift together with the head 61 will be described. Figs. 8A to 8D are views each illustrating a specific example of the horizontal shifting of the tip 10. An upper row in each drawing shows a plan view of the leading end portion 10T seen in an axial direction of the tip 10, and a lower row shows a cross-sectional view of the leading end portion 10T along the axial direction. Figs. 8A to Fig. 8D each illustrates an example of a linear shifting of the leading end portion 10T in an X-direction (horizontal direction), the linear shifting falling within a range of a width of the leading end portion 10T. In the example, a cell C to be peeled off has a size smaller than a diameter of the leading end opening t of the leading end portion 10T.

Fig. 8A illustrates a state where the leading end portion 10T of the tip 10 having a cylindrical shape and the leading end opening t is aligned with the cell C to be sucked. Specifically, the leading end portion 10T comes into contact with the bottom surface 31 in a state where a target point O of the cell C defined around the center in a plan view meets an opening center t0 of the leading end opening t. The leading end portion 10T has a circular cylindrical body with a radius r. In other words, the width (outer diameter) of the leading end portion 10T is denoted by 2r. The cell C falls within a range of the leading end opening t. Here, the position of the target point O in the X-direction is defined as a point p0 or a predetermined origin position.

Fig. 8B illustrates a state where the opening center t0 is located at a point P1 after shifting from the point p0 in the -X-direction or first horizontal direction by a distance of a radius r of the leading end portion 10T. In the shifting to the point p1, the cell C is pushed by the inner wall of the leading end portion 10T, and an +X-side portion of the cell C is consequently peeled off from the bottom surface 31. Thereafter, the leading end portion 10T is oriented in the horizontally opposite direction to shift in the +X-direction or second horizontal direction.

Fig. 8C illustrates a state where the opening center t0 is located at a point P2 after leaving the point p1, passing through the point P0 at the origin position, and shifting in the +X-direction by 2r corresponding to a distance of the diameter of the leading end portion 10T. In the shifting from the point p1 to the point p2, the inner wall of the leading end portion 10T peels off an -X-side portion of the cell C remaining adhered to the bottom surface 31. In other words, this shifting results in scraping away the cell C along the whole surface thereof from the bottom surface 31.

Fig. 8D illustrates a state where the opening center t0 returns to the point P0 after shifting from the point p2 in the -X-direction or first horizontal direction by a distance of the radius r of the leading end portion 10T. In this state, the cell C stays away from the inner wall of the leading end portion 10T, and the cell C is located around the opening center t0. Consequently, the cell C can be easily sucked into the tip 10.

As illustrated in Fig. 8A, when the height position of the bottom surface 31 where the cell C is adhered meets a lowered position of the leading end portion 10T, execution of only one linear shifting in a reciprocating manner within a range of the width of the leading end portion 10T enables peeling-off of the cell C from the bottom surface 31. In other words, a minimum shifting of the head 61 attains peeling-off of the cell C falling within the range of the width of the leading end portion 10T. Even when the height position of the bottom surface 31 and the current lowered position of the leading end portion 10T do not meet, another linear shifting in a reciprocating manner at a different stage position along the vertical direction enables the peeling-off of the cell C from the bottom surface 31.

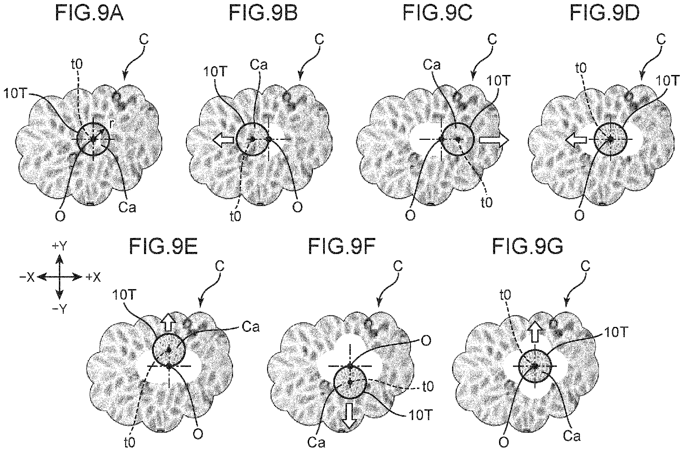

Figs. 9A to 9G are views illustrating an example of a peeling-off operation when the cell C has a size larger than the diameter of the leading end opening t of the leading end portion 10T. Each drawing illustrates a specific example of a horizontal shifting of the tip 10 in a case where a cell fragment Ca is partly peeled off from the cell C in the form of a colony. Fig. 9A illustrates a state where the leading end portion 10T of the tip 10 and the cell fragment Ca to be sucked are aligned. Specifically, the leading end portion 10T is in surface contact with the bottom surface 31 in a state where a target point O determined on the cell C for sucking the cell fragment Ca meets the opening center t0 of the leading end opening t. Thereafter, the leading end portion 10T linearly shifts in a cross shape centered on the target point O.

Fig. 9B illustrates a state of the leading end portion 10T shifted in such a manner that the opening center t0 is away from the target point O in the -X-direction by a distance of the radius r of the leading end portion 10T. Subsequently, the leading end portion 10T is shifted in such a manner that the opening center t0 shifts in the +X-direction by the distance 2r, as illustrated in Fig. 9C. Thereafter, the leading end portion 10T is shifted in such a manner that the opening center t0 returns in the -X-direction by the distance r to reach the target point O, as illustrated in Fig. 9D. Heretofore, the shifting operation is similar to that illustrated in Figs. 8A to 8D.

Then, the leading end portion 10T is shifted in such a manner that the opening center t0 shifts in the +Y-direction by a distance r, as illustrated in Fig. 9E. Subsequently, the leading end portion 10T is shifted in such a manner that the opening center t0 shifts in the -Y-direction by a distance 2r, as illustrated in Fig. 9F. The leading end portion 10T is then shifted in such a manner that the opening center t0 returns in the +Y-direction by the distance r to reach the target point O, as illustrated in Fig. 9G. The leading end portion 10T having executed the above-described horizontal shifting succeeds in reliably peeling off the partial cell fragment Ca from the cell C which is larger than the leading end portion 10T.

Meanwhile, a technology of causing protein or a nucleus of the cell C to fluoresce by employing a fluorescent process is widely adopted. The cell fragment Ca to be peeled off in the example illustrated in Figs. 9A to 9G may be a specific portion of the cell C to fluoresce when subjected to the fluorescent process.

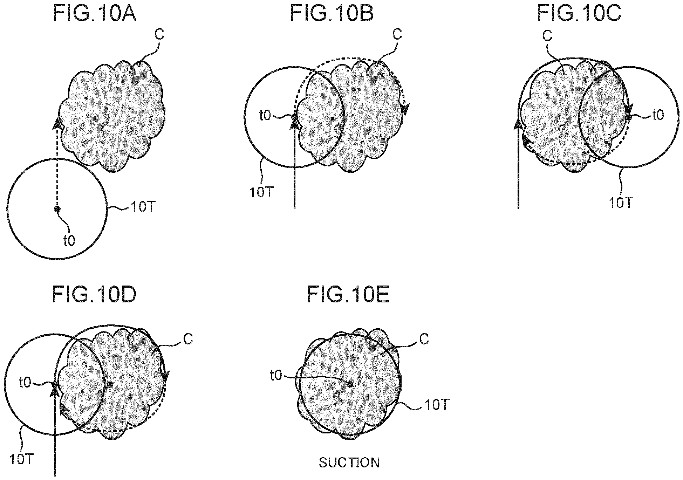

Figs. 10A to 10E are views each illustrating another example of the horizontal shifting of the tip 10 in an operation of peeling off the cell C. A cultured cell C generally grows in an approximately circular shape in a plan view in many cases. Shown here is an example where the shifting containing a horizontal component as executed by the leading end portion 10T of the tip 10 indicates a curvy shifting drawing a circle. The curvy shifting may include a shifting drawing a swirl. Figs. 10A to 10E illustrate an example of the curvy shifting of the leading end portion 10T along a peripheral edge of the cell C.

Fig. 10A illustrates a state where the leading end portion 10T is arranged in the vicinity of a cell C to be a target. The leading end portion 10T and the cell C do not come into contact with each other at this position yet. Thereafter, the leading end portion 10T is horizontally shifted in such a manner that the opening center t0 approaches the peripheral edge of the cell C. Fig. 10B illustrates a state where the opening center t0 has reached the peripheral edge of the cell C. At this time, when the height position of the leading end portion 10T exists such a height position as to come into contact with the cell C, an outer wall of the leading end portion 10T pushes the cell C to peel off a part of the cell from the bottom surface 31.

Fig. 10C illustrates a state of the leading end portion 10T curvingly shifted until the opening center t0 reaches a position at a semicircular distance along the peripheral edge of the cell C. Fig. 10D illustrates a state where the opening center t0 is further shifted at a remaining semicircular distance along the peripheral edge of the cell C, resulting in shifting in one circle. Consequently, the peeling-off of the cell C is completed. Fig. 10E illustrates a state where the peeled-off cell C is sucked by the tip 10. In this case, the tip 10 is once lifted from the state illustrated in Fig. 10D to approximately align the opening center t0 with the center position of the cell C. Thereafter, the tip 10 is lowered, if necessary, to execute an action of sucking the cell C. According to the horizontal shifting, the peripheral edge of the cell C is preferentially peeled off from the bottom surface 31, and thus, the ability of peeling off the cell C can be increased.

### [Operation sequence of peeling-off control]

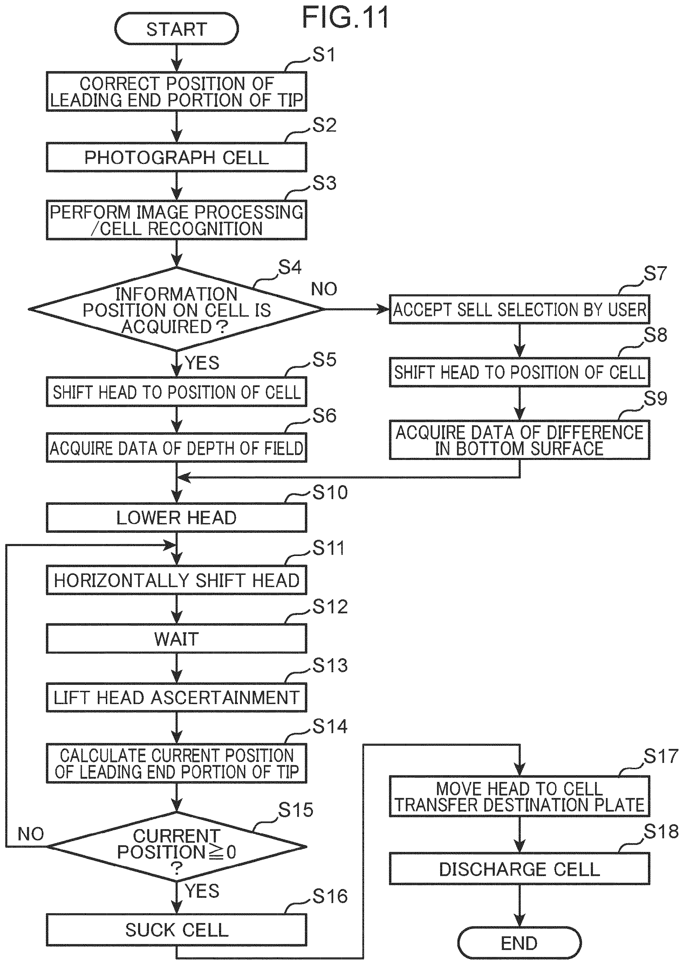

Fig. 11 is a flowchart showing picking and transferring of a cell C by using the cell transferring device S. Here, the tip 10 is attached to the head 61 and the cell C which has been cultured is adhered to the bottom surface 31 of the well 3 of the culture plate 2 before a start of the relevant process. The control of gradually shifting the leading end portion 10T away from the bottom surface 31 is adopted as the peeling-off control is as exemplified in Fig. 6A.

With reference to Fig. 3 as well, once the process is started, the main control part 78 causes the camera unit 5 to photograph the tip 10 attached to the head 61, and executes, on the basis of an obtained image, a correction processing of a position of the leading end portion 10T of the tip 10 (step S1). This correction is intended for solving possible deviation of the leading end portion 10T from a predetermined reference position due to, for example, a misalignment in the attachment of the tip 10 to the head 61.

Subsequently, the main control part 78 causes the camera unit 5 to photograph the cell C adhered to the bottom surface 31 of the well 3 (step S2). Acquired image data is transmitted to the image processing part 74 to be subjected to predetermined image processing like pattern recognition, and a two-dimensional shape of the cell C is recognized. The cell position specifying section 781 then executes acquisition of position information about coordinate data indicating an XYZ position of the recognized cell C (step S3).

Subsequently, it is confirmed whether the position information on the cell C is acquired (step S4). When the position information on the cell C is acquired (YES in step S4), the main control part 78 causes the head unit axis drive part 63 to shift the head unit 6 through the axis control part 71 so that the head 61 or the tip 10 comes above the cell C to be transferred (step S5). Thereafter, the picking control section 782 acquires depth of field data of the lens section 51 of the camera unit 5 from the storage part 75 (step S6).

When the position information on the cell C is not acquired (NO in step S4), the main control part 78 accepts a cell selection by a user from the input part 76 (step S7). The user selects a cell C to be transferred and inputs the selection to the input part 76 while visually confirming the display part 77. Further, the main control part 78 causes the head 61 or the tip 10 to come above the cell C selected to be transferred (step S8). Thereafter, the picking control section 782 acquires data of the height difference in the bottom surface 31 from the storage part 75 (step S9).

After step S6 or step S9, the picking control section 782 controls the head lifting and lowering motor 641 to lower the head 61 (step S10). In the subsequence from step S6 in the flowchart, the head 61 is lowered by a distance obtained by adding a depth of field to the height position of the cell C obtained through image recognition. In the subsequence from step S9 in the flowchart, the head 61 is lowered by a distance obtained by adding the height difference in the bottom surface 31 acquired in step S9 to a predetermined height position of the bottom surface 31. The height position of the leading end portion 10T of the tip 10 at this time is defined as a "current position" along the vertical direction.

The picking control section 782 controls the head unit axis drive part 63 to horizontally shift the head 61 at the "current position" (step S11). The horizontal shift includes, for example, a linear shifting in the XY-directions for scraping away the cell C adhered to the bottom surface 31 by the leading end portion 10T. Subsequently, the picking control section 782 executes a wait of suspending the operation of the head 61 for a predetermined time (step S12). The waiting indicates a pose time for waiting restoration of the tip 10 from possible deformation attributed to the horizontal shifting.

The picking control section 782 then controls the head lifting and lowering motor 641 to lift the head 61 by a predetermined unit pitch (d1 in Fig. 6A) (step S13). Subsequently, the picking control section 782 obtains a height position of the leading end portion 10T after the lifting, and the obtained height position is defined as the "current position" (step S14). It is confirmed whether the "current position" exceeds a point of a height difference = 0 (step S15).

For instance, in the subsequence from step S6 in the flowchart, the depth of field is presumed to indicate -30 µm. In this case, the "current position" of the leading end portion 10T in step S10 means a height position of the recognized cell C at -30 µm. When the lifting pitch is defined as d1 = 10 µm in step S13, the leading end portion 10T is at the "current position" of-20 µm after the finish of the first lifting of the head 61. Here, the condition "current position ≧ 0" is not satisfied (NO in step S15), and hence, the process returns to step S11 to execute a horizontal shifting of the head 61 second time. Thereafter, a similar shifting is repeated until the condition "current position ≧ 0" is satisfied, i.e., the current position corresponds to the depth of field of 30 µm.

By contrast, when the condition "current position ≧ 0" is satisfied (YES in step S15), the picking control section 782 controls the plunger lifting and lowering motor 642 to generate a suction force at the leading end opening t of the leading end portion 10T to suck the cell C into the tip 10 (step S16). Thereafter, the main control part 78 controls the head unit axis drive part 63 to move the head 61 together with the head unit 6 to the position of the destination plate 4 (step S17). The main control part 78 then controls the plunger lifting and lowering motor 642 to discharge the cell C held in the tip 10 into a target well 41 (step S18).

### [Another embodiment]

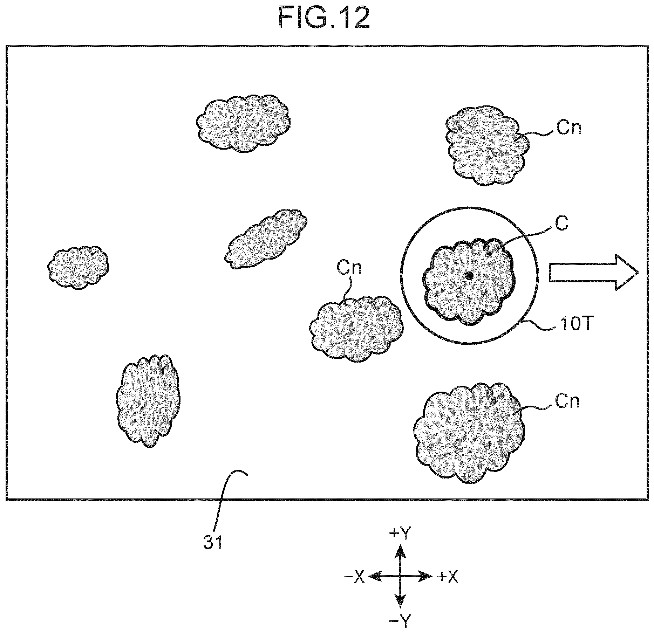

Fig. 12 is a view illustrating another example of an operation of peeling off a cell C. The example illustrated in Fig. 12 shows a peeling-off operation suitable for a case where many cells C are adhered to a bottom surface 31 of a well 3. One cell C on the bottom surface 31 is selected to be picked. A leading end portion 10T of a tip 10 is aligned with the cell C, and, for example, the leading end portion 10T is horizontally shifted in XY-directions to peel off the cell C. However, in the example illustrated in Fig. 12, another cell Cn exists around the cell C to be picked. In this case, the leading end portion 10T may damage another cell Cn which is not a target or suck the cell Cn along with a horizontal shifting thereof.

In such a case, a main control part 78 may acquire, on the basis of an image of the bottom surface 31 photographed by a camera unit 5, distribution information on the cells C, Cn on the bottom surface 31. A picking control section 782 can set, on the basis of the distribution information, a shifting direction of a head 61 in a peeling-off control.

In the example illustrated in Fig. 12, other cells Cn exist in the +Y-direction, the -X-direction, and the -Y-direction in view of the cell C, but no cell Cn exists in the +X-direction. In the cell distribution, the leading end portion 10T or the head 61 is shifted in the +X-direction in the peeling-off control. This shifting enables picking of the target cell C without damage to other cells Cn existing on the bottom surface 31.

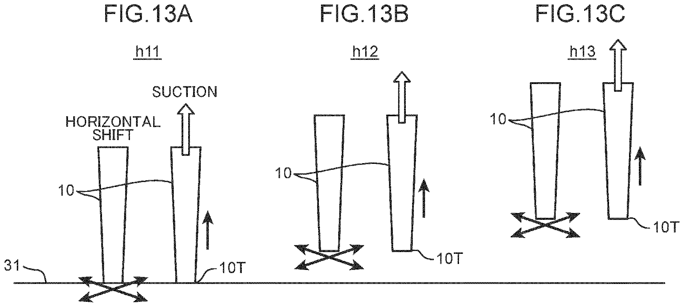

Figs. 13A to 13C are views illustrating another example of an operation of peeling off the cell C. In the example illustrated here, an action of sucking the cell C into the tip 10 is executed in horizontally shifting the head 61 at different positions along the vertical direction, i.e., executed at each height position along the vertical direction.

Fig. 13A illustrates a state where the leading end portion 10T is arranged at a first position h11 and the horizontal shifting of the tip 10 is executed. Thereafter, the tip 10 or the head 61 executes the action of sucking the cell C at the first position h11. Fig. 13B illustrates a state where the leading end portion 10T is arranged at a second position h12 which is higher than the first position h11. A horizontal shifting and the suction action of the tip 10 are executed at the second position h12 as well. Fig. 13C illustrates a state where the leading end portion 10T is arranged at a third position h13 which is further higher than the second position h12. A horizontal shifting and the suction action of the tip 10 are executed at the third position h13 as well.

According to each of the examples in Fig. 13A to 13C, the action of sucking the cell C into the tip 10 is executed while the peeling-off control is executed. Execution of the action of sucking the cell after completion of a series of the peeling-off control may make the cell C peeled off from the bottom surface 31 float, and thus may result in a failure to suck the cell into the tip 10. Alternatively, the cell C peeled-off along with a horizontal shifting of the tip 10 at a lower stage may be scattered or dispersed in another horizontal shifting thereof at an upper stage. However, such execution of the suction action at each stage along the vertical direction enhances a success rate of sucking the cell C. It is noted here that the suction action can serve to gradually lift a plunger 12 at each stage along the vertical direction.

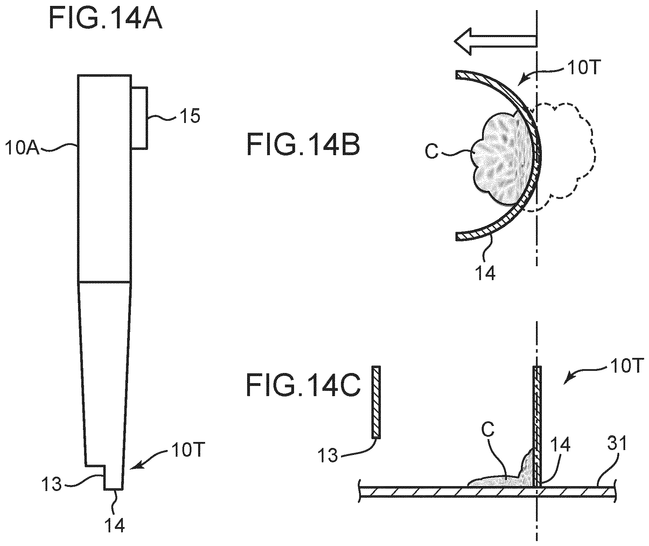

Next, a modification of the tip 10 will be described. Fig. 14A illustrates a tip 10A according to the modification. The tip 10A has a notch 13 and a contact section 14 in the leading end portion 10T. The contact section 14 represents a section of the leading end portion 10T that is contactable with a bottom surface 31. The notch 13 is recessed from the contact section 14 in the vertical direction, that is, recessed upward by around a half of the leading end portion 10T. The tip 10A further has a positioning protrusion 15 having a rod shape and protruding from the peripheral surface of the tip. The positioning protrusion 15 is provided to align a rotation origin of the head 61 which rotates about an R-axis with a position of the notch 13.

Fig. 14B is a horizontal cross-sectional view of the leading end portion 10T and illustrates a cell peeling-off operation using the tip 10A. Fig. 14C is a vertical cross-sectional view of the leading end portion 10T. In the peeling-off of the cell C, a lower end surface of the contact section 14 comes into contact with the bottom surface 31. The notch 13 enables the contact section 14 to serve as a scraper. Along with the horizontal shifting of the tip 10A, the contact section 14 pushes the cell C adhered to the bottom surface 31 in the direction denoted by the arrow in the drawing. In this manner, the cell C is peeled off from the bottom surface 31. When a pressing force of the leading end portion 10T against the bottom surface 31 is high, the contact section 14 is curved and deformed to have a large frictional force, and thus, the ability of peeling off the cell C is further increased.

The notch 13 serves to avoid contact with another cell C except for a target cell. Specifically, when the head 61 and the tip 10A horizontally shift together to peel off the target cell C, the notch 13 makes it possible to avoid contact between another adjacent cell C and the leading end portion 10T while passing by the adjacent cell C. The notch 13 is further available as a suction hole. A leading end portion 10T without the notch 13 may fail to execute the suction action when the leading end portion 10 comes into close contact with the bottom surface 31. By contrast, in the tip 10A, the notch 13 remains as an open region even in a state where the leading end portion 10T is in contact with the bottom surface 31, and thus, the tip 10A can execute the action of sucking the cell C.

In the cell transferring device S according to the embodiment as described heretofore, the picking control section 782 controls, in the peeling-off control of the cell C, the shifting of the head 61 in such a manner that the head 61 performs a shifting containing a horizontal component at each of different stage positions along the vertical direction. In the horizontal shifting, the leading end portion 10T of the tip 10 comes into contact with the cell C adhered to the bottom surface 31 of the well 3 at least at any one of the different stage positions. This consequently achieves a great increase in the probability of peeling off the cell C by the leading end portion 10T even when the bottom surface 31 has irregularities or protrusions and recesses, or the height position of the cell C involves an error attributed to the depth of field.

### [Invention included in the embodiment]

A cell transferring device according to one aspect of the present invention includes: a head that is to be attached with a tip including a leading end portion having an opening for allowing a cell to enter and leave the tip, includes a mechanism for sucking and discharging the cell into and from the tip through the opening, and is shiftable in three-dimensional directions; and a controller that controls a shifting of the head. The controller executes a peeling-off control of causing the tip to peel off the cell adhered to a bottom surface of a cell container through the head. The controller controls, in the peeling-off control, the head in such a manner that the leading end portion of the tip performs a shifting containing a horizontal component at each of different positions along a vertical direction, the different positions including a position where the leading end portion of the tip comes into contact with the cell adhered to the bottom surface.

According to the cell transferring device, the controller can control, in the peeling-off control, the shifting of the head in such a manner that the head performs a shifting containing a horizontal component at each of the different positions along the vertical direction. In this manner, the tip leading end portion comes into contact with the cell adhered to the bottom surface of the cell container at least at any one of the different positions owing to the shifting, even when the bottom surface has irregularities or protrusions and recesses, or an error occurs in specifying a height position of the cell. Alternatively, even when the bottom surface has the irregularities or protrusions and recesses, the leading end portion of the tip comes into contact with the cell adhered to a certain protrusion or recess on the bottom surface at each different position along the height direction of the protrusion or recess. This results in achieving a great increase in the probability of peeling off the cell adhered to the bottom surface by the leading end portion.

In the cell transferring device, the shifting containing the horizontal component desirably includes a first horizontal shifting of shifting the head in a horizontal direction at a first position along the vertical direction, and a second horizontal shifting of shifting the head in a horizontal direction at a second position different from the first position along the vertical direction.

According to the cell transferring device, a simple shifting of the head in a horizontal direction realizes the "shifting containing the horizontal component" by the head. The shifting control of the head thus can be simplified.

In the cell transferring device, it is desirable that the second position is located away from the bottom surface of the cell container further than the first position, and that the controller causes the head to perform the first horizontal shifting and subsequently perform the second horizontal shifting.

According to the cell transferring device, first, the first horizontal shifting is performed at the first position closer to the bottom surface, and subsequently, the second horizontal shifting is performed at the second position further away from the bottom surface than the first position. In other words, after the first horizontal shifting, the head is shifted away from the bottom surface in the vertical direction to perform the second horizontal shifting. For instance, setting of the first position to a position where the leading end portion of the tip comes into contact with the bottom surface leads to achieved execution of the first horizontal shifting in the state where the leading end portion is ensured to be in contact with the cell. Besides, the contact position with the bottom surface serves as the first position. This facilitates setting of a shifting pitch to the second position. Consequently, the picking of the cell is speedily executable.

In the opposite way, the second position may be located closer to the bottom surface of the cell container than the first position, and the controller may cause the head to perform the first horizontal shifting and subsequently perform the second horizontal shifting.

According to the cell transferring device, after the first horizontal shifting, the head is shifted closer to the bottom surface to perform the second horizontal shifting. This hence permits the head to horizontally shift while causing the leading end portion of the tip to gradually approach the cell adhered to the bottom surface.

In the cell transferring device, the controller desirably shifts the head in such a manner that a shifting locus of the leading end portion in the first horizontal shifting and a shifting locus of the leading end portion in the second horizontal shifting intersect on an axially viewed plane of the tip.

According to the cell transferring device, the shifting locus of the leading end portion differs between the first horizontal shifting and the second horizontal shifting. Thus, directionally different peeling-off forces are generated to act on the cell adhered to the bottom surface, resulting in achievement of further easier peeling-off of the cell.

In the cell transferring device, the shifting containing the horizontal component desirably further includes a third horizontal shifting of shifting the head in a horizontal direction at a third position different from the first position and the second position along the vertical direction.

The cell transferring device achieves execution of the third horizontal shifting in addition to the first horizontal shifting and the second horizontal shifting. Specifically, the horizontal shifting performable at more positions along the vertical direction can facilitate the peeling-off of the cell from, for example, the bottom surface even having large irregularities or protrusions and recesses.

In the cell transferring device, it is desirable that the shifting containing the horizontal component includes a horizontally linear shifting, and the controller controls the head to perform the horizontally linear shifting within a range of a width of the leading end portion of the tip.

The cell transferring device succeeds in peeling off the cell falling within the width of the leading end portion by a minimum shifting of the head.

In this case, it is desirable that the leading end portion of the tip is a cylindrical leading end portion with the opening having a circular shape, and the controller controls the shifting of the head in such a manner that the center of the opening in the circular shape sequentially: shifts from a predetermined origin position in a first horizontal direction by a distance of a radius of the cylindrical leading end portion; passes through the origin position, and shifts in a second horizontal direction opposite to the first horizontal position by a distance of a diameter of the cylindrical leading end portion in the circular shape; and shifts in the first horizontal direction by a distance of the radius of the cylindrical leading end portion in the circular shape to return to the origin position.

The cell transferring device succeeds in peeling off the cell falling within a range of the leading end portion in the circular shape by a minimum shifting in a reciprocating manner from the origin position in the first and second horizontal directions.

In the cell transferring device, it is desirable that the shifting containing the horizontal component includes a curvy shifting drawing a circle or a swirl on a horizontal plane, and that the controller controls the shifting of the head in a such manner that the leading end portion of the tip curvingly shifts along a peripheral edge of the cell.

In many cases, a cell colony is adhered to the bottom surface in a substantially circular shape in a top view. According to the cell transferring device, the peripheral edge of the cell is preferentially peeled off from the bottom surface, and thus, the ability of peeling off the cell from the bottom surface can be increased.

In the cell transferring device, the controller desirably acquires distribution information on cells on the bottom surface of the cell container, and sets a shifting direction of the head in the peeling-off control on the basis of the distribution information.

The cell transferring device achieves picking of the target cell without damage to other cells existing on the bottom surface.

In the cell transferring device, the different positions along the vertical direction desirably includes a position where the leading end portion of the tip comes into contact with the bottom surface of the cell container.

According to the cell transferring device, the position where the leading end portion comes into contact with the bottom surface is defined as a reference position, and then, a position along the vertical position where the horizontal shift of head is performed can be determined on the basis thereof. That is to say, the position of the leading end portion is rapidly determinable, and picking of the cell is efficiently executable.

In the cell transferring device, it is desirable that the controller is configured to control the mechanism included in the head for the sucking and the discharging, and that the controller causes the head to execute an action of sucking the cell while causing the head to horizontally shift at each of the different positions along the vertical direction in the peeling-off control.

According to this cell transferring device, the action of sucking the cell into the tip is executed while the peeling-off control is executed. Execution of the action of sucking the cell after completion of the peeling-off control may make the cell peeled off from the bottom surface float, and thus may result in a failure to suck the cell into the tip. The aforementioned configuration contributes to an increase in a success rate of the cell suction.

In the cell transferring device, the leading end portion of the tip desirably has a contact section that comes into contact with the bottom surface of the cell container, and a notch recessed from the contact section in the vertical direction.

According to the cell transferring device, when the head and the tip horizontally shift together to peel off the target cell, the notch makes it possible to avoid contact with another adjacent cell. The notch further remains as an open region even in a state where the leading end portion is in contact with the bottom surface, and thus, the tip can execute the sucking action.

In the cell transferring device, at least one of the tip or the cell container is desirably made of an elastically deformable member.

According to the cell transferring device, at least one of the tip or the cell container elastically deforms even when the leading end portion of the tip relatively strongly comes into contact with the bottom surface of the cell container. This leads to a reduction in the risk of damage to these members. Moreover, a strong sliding frictional force (frictional force) based on the elastic deformation force can act between the tip and the surface, and therefore, the force of peeling off the cell from the bottom surface can be increased.

Conclusively, the present invention described heretofore can provide a cell transferring device that enables accurate picking of a cell by reliably peeling off the cell from a bottom surface of a container.

## Claims

1. A cell transferring device, comprising:
a head that is to be attached with a tip including a leading end portion having an opening for allowing a cell to enter and leave the tip, includes a mechanism for sucking and discharging the cell into and from the tip through the opening, and is shiftable in three-dimensional directions; and
a controller that controls a shifting of the head, wherein
the controller is configured to:
execute a peeling-off control of causing the tip to peel off the cell adhered to a bottom surface of a cell container through the head; and
control, in the peeling-off control, the head in such a manner that the leading end portion of the tip performs a shifting containing a horizontal component at each of different positions along a vertical direction, the different positions including a position where the leading end portion of the tip comes into contact with the cell adhered to the bottom surface.

2. The cell transferring device according to claim 1, wherein the shifting containing the horizontal component includes a first horizontal shifting of shifting the head in a horizontal direction at a first position along the vertical direction, and a second horizontal shifting of shifting the head in a horizontal direction at a second position different from the first position along the vertical direction.

3. The cell transferring device according to claim 2, wherein the second position is located away from the bottom surface of the cell container further than the first position, and
the controller causes the head to perform the first horizontal shifting and subsequently perform the second horizontal shifting.

4. The cell transferring device according to claim 2, wherein the second position is located closer to the bottom surface of the cell container than the first position, and
the controller causes the head to perform the first horizontal shifting and subsequently perform the second horizontal shifting.

5. The cell transferring device according to any one of claims 2 to 4, wherein the controller shifts the head in such a manner that a shifting locus of the leading end portion in the first horizontal shifting and a shifting locus of the leading end portion in the second horizontal shifting intersect on an axially viewed plane of the tip.

6. The cell transferring device according to any one of claims 2 to 5, wherein the shifting containing the horizontal component further includes a third horizontal shifting of shifting the head in a horizontal direction at a third position different from the first position and the second position along the vertical direction.

7. The cell transferring device according to any one of claims 1 to 6, wherein the shifting containing the horizontal component includes a horizontally linear shifting, and
the controller controls the head to perform the horizontally linear shifting within a range of a width of the leading end portion of the tip.

8. The cell transferring device according to claim 7, wherein the opening of the leading end portion of the tip has a circular shape, and
the controller controls the shifting of the head in such a manner that the center of the opening in the circular shape sequentially:
shifts from a predetermined origin position in a first horizontal direction by a distance of a radius of the opening in the circular shape;
passes through the origin position, and shifts in a second horizontal direction opposite to the first horizontal position by a distance of a diameter of the opening in the circular shape; and
shifts in the first horizontal direction by a distance of the radius of the opening in the circular shape to return to the origin position.

9. The cell transferring device according to any one of claims 1 to 6, wherein the shifting containing the horizontal component includes a curvy shifting drawing a circle or a swirl on a horizontal plane, and
the controller controls the shifting of the head in a such manner that the leading end portion of the tip curvingly shifts along a peripheral edge of the cell.

10. The cell transferring device according to any one of claims 1 to 6, wherein the controller acquires distribution information on cells on the bottom surface of the cell container, and sets a shifting direction of the head in the peeling-off control on the basis of the distribution information.

11. The cell transferring device according to any one of claims 1 to 10, wherein the different positions along the vertical direction include a position where the leading end portion of the tip comes into contact with the bottom surface of the cell container.

12. The cell transferring device according to any one of claims 1 to 11, wherein the controller is configured to control the mechanism included in the head for the sucking and the discharging, and
the controller causes the head to execute an action of sucking the cell while causing the head to horizontally shift at each of the different positions along the vertical direction in the peeling-off control.

13. The cell transferring device according to any one of claims 1 to 12, wherein the leading end portion of the tip has a contact section that comes into contact with the bottom surface of the cell container, and a notch recessed from the contact section in the vertical direction.

14. The cell transferring device according to any one of claims 1 to 12, wherein at least one of the tip or the cell container is made of an elastically deformable member.
